# FASCICULE DE BREVET EUROPEEN

(11) **EP 3 478 648 B1**
(45) Date de publication et mention de la délivrance du brevet: **17.06.2020**
(21) Numéro de dépôt: 17731604.9
(22) Date de dépôt: 26.06.2017
(51) Int. Cl.: C07C 7/11, C07C 7/00, C07C 1/20, C07C 7/04, C07C 7/08, C07C 11/167

(54) **PROCEDE DE PRODUCTION DE BUTADIENE A PARTIR D'ETHANOL COMPRENANT UNE PURIFICATION D'UN EFFLUENT RICHE EN BUTADIENE PAR DISTILLATION EXTRACTIVE**
VERFAHREN ZUR HERSTELLUNG VON BUTADIEN AUS ETHANOL MITTELS REINIGUNG EINES BUTADIENREICHEN ABWASSERS DURCH EXTRAKTIVE DESTILLATION
METHOD FOR THE PRODUCTION OF BUTADIENE FROM ETHANOL, COMPRISING THE PURIFICATION OF A BUTADIENE-RICH EFFLUENT BY MEANS OF EXTRACTIVE DISTILLATION

(30) Priorité: 29.06.2016 FR 1656050
(43) Date de publication de la demande: 08.05.2019
(73) Titulaire: IFP Energies nouvelles, 92852 Rueil-Malmaison (FR); Compagnie Générale des Etablissements Michelin, 63000 Clermont-Ferrand (FR)
(72) Inventeur: COUDERC, Sophie, 92200 Neuilly Sur Seine (FR); DASTILLUNG, Rejane, 69005 Lyon (FR); THINON, Olivier, 42300 Roanne (FR)
(86) Numéro de dépôt international: PCT/EP2017/065741
(87) Numéro de publication internationale: WO 2018/001981

(56) Documents cités:
- WO-A1-2015/079040
- WO-A1-2016/042095

## Description

### DOMAINE TECHNIQUE DE L'INVENTION

L'invention concerne un procédé de production de butadiène à partir d'éthanol.

### ART ANTÉRIEUR

Le procédé de production de 1,3-butadiène à partir d'éthanol, en une ou deux étape(s) réactionnelle(s), a un taux de conversion par passe limité. Ceci induit des recyclages importants, rendus complexes par le grand nombre d'impuretés co-produites avec le 1,3-butadiène, dont l'extraction obère le rendement global du procédé. Chaque perte dans les opérations unitaires, en particulier dans les nombreuses opérations de séparation, se traduit donc par une perte globale au sein du procédé qui devient rapidement inacceptable d'un point de vue économique. Ces pertes ont conduit à l'arrêt d'exploitation de ces procédés à l'issue de la seconde guerre mondiale.

Parmi les impuretés, on peut citer des hydrocarbures comprenant de 1 à 16 atomes de carbone, saturés ou insaturés, voire aromatiques, ainsi que des produits oxygénés tels que des alcools, des phénols, des aldéhydes, des cétones, des acides, des esters, des éthers, des acétals, saturés ou insaturés, voire aromatiques.

Dans les conditions normales de température et de pression, on peut citer parmi les sous-produits gazeux principaux l'hydrogène, le monoxyde de carbone, le dioxyde de carbone, les alcanes et oléfines en C₁-C₄ et le méthyle éthyle éther, et parmi les sous-produits liquides principaux les pentènes, les pentadiènes, le diéthyl éther, l'éthyle vinyl éther, les hexènes, les hexadiènes, le butanal, le crotonaldéhyde, l'acétate d'éthyle, le diéthyle acétal, le butanol, l'hexanol, l'acide acétique.

D'autres sous-produits sont générés en quantité infimes. Dans la suite du document, on parlera d' « huiles brunes » pour designer l'ensemble de centaines de composés hydrocarbures et oxygénés produits dans les sections réactionnelles dont les températures d'ébullition sont comprises entre celle de l'éthanol et allant jusqu'à 600°C. Ces huiles brunes ont la particularité d'être solubles dans l'éthanol, mais insolubles dans l'eau. Elles peuvent, partout où elles ne sont pas diluées par un fort excès d'éthanol, encrasser et boucher les équipements. Par ailleurs, ces huiles brunes sont problématiques au sein de la colonne à distiller qui sépare l'eau produite par la réaction et l'éthanol non converti. En effet, ces huiles brunes sont solubles dans l'effluent eau-éthanol alimentant ladite colonne à distiller, et insolubles dans le résidu essentiellement constitué d'eau. Une séparation de phase se produit donc au sein de cette colonne, diminuant considérablement l'efficacité de la séparation. Les huiles brunes sont difficiles à éliminer au sein du procédé du fait qu'elles sont constituées de centaines de composés ayant des propriétés physico-chimique très différentes. Une fraction de ces huiles brunes s'accumule donc au sein du procédé, entrainant une baisse de son efficacité au bout de quelques jours, au mieux semaines, d'opération et nécessitant de purger périodiquement certains flux. La perte en éthanol et en acétaldéhyde engendrée dégrade le rendement global du procédé pour un coût qui serait aujourd'hui rédhibitoire.

La purification du butadiène fait appel à une combinaison de nombreuses opérations unitaires, telles que des lavages, des distillations simples et extractives. L'art antérieur enseigne l'utilisation de distillations extractives mettant en oeuvre un solvant bis(2-chloroéthyle)éther, ou Chlorex, aujourd'hui proscrit car très toxique. Il est important de noter que les spécifications du butadiène sont aujourd'hui extrêmement sévères, de par la sensibilité des catalyseurs de polymérisation du butadiène. Par exemple, la spécification en acétaldéhyde (réactif intermédiaire permettant de produire le butadiène) dans le butadiène est passée de 1000 ppm à moins de 10 ppm aujourd'hui. L'ouvrage « Synthetic rubber from Alcohol», (A. Talalay, M. Magat, 1945) donne une vue générale des procédés développés jusque dans les années 40.

Le brevet US 2,409,250 décrit les étapes de purification successives du butadiène (extraction, première purification et ultime purification du butadiène par super-fractionnement). Le butadiène est produit à une pureté de 98,7%, mais au prix d'une perte significative de rendement. Pour limiter cette perte, les produits de têtes de la colonne de purification du butadiène par super-fractionnement sont soutirés et en partie recyclés vers l'étape d'extraction du butadiène. Ces recyclages importants, en particulier le recyclage du flux butène/butadiène en vue d'éliminer les incondensables, induisent un surdimensionnement des équipements.

Le brevet US 1,948,777 décrit dans le détail l'étape d'ultime purification du butadiène par distillation extractive mettant en oeuvre différents solvants, dont le Chlorex. En limitant la perte en butadiène en tête de colonne, soit une concentration de 0,2% de butadiène dans la distillat, la pureté du butadiène obtenu en fond n'est que de 70%, alors qu'en cherchant à obtenir un butadiène plus pur en fond, soit 99%, la perte en butadiène en tête est beaucoup plus importante, avec une concentration de butadiène dans le distillat de 30%. La production d'un butadiène de haute pureté est donc réalisée au prix d'une très forte baisse de rendement global de l'unité.

Le document WO14199348 décrit une méthode d'obtention du butadiène à partir d'un effluent contenant de l'éthanol et éventuellement de l'acétaldéhyde sur un catalyseur à base d'un matériau zéolitique. Des conversions d'éthanol supérieures à 95 % et des sélectivités en butadiène entre 20 et 48 % sont obtenues. Le brevet mentionne la production de composés oxygénés tel que le diéthyl éther, le crotonaldéhyde et l'éthylacétate qui sont séparés du butadiène par distillation sans qu'il soit donné plus de détails. Aucune information n'est fournie sur la gestion des nombreuses autres impuretés pouvant être présentes et sur les moyens de purification du butadiène afin de répondre aux spécifications requises quant à son utilisation dans les procédés avals.

Le catalyseur se désactive au cours de son utilisation avec pour conséquence une dégradation de la sélectivité en butadiène et la production en plus grande quantité d'impuretés telles que les butyne-1, le 1,2-butadiène, le n-butane et les butènes. Ces impuretés se retrouvent partiellement ou totalement entraînées avec le butadiène dans les étapes permettant de séparer celui-ci de l'acétaldéhyde et l'éthanol.

Les documents WO2016/042096 et WO2016/042095 décrivent un procédé de production de butadiène à partir d'une charge éthanol, respectivement en 1 et 2 étapes réactionnelles, avec un agencement d'opérations unitaires permettant d'éliminer les impuretés gazeuses et liquides tout en minimisant la perte en éthanol et acétaldéhyde, améliorant ainsi le rendement global de l'unité tout en réduisant le flux global d'eau nécessaire aux étapes de séparation et en obtenant un butadiène très pur. La purification finale du butadiène est réalisée par extraction liquide-liquide. Cependant, des impuretés spécifiques tel que les butynes peuvent être produites dans des teneurs critiques au regard de la spécification du produit butadiène obtenu par le procédé lorsque les catalyseurs se désactivent. Ce problème n'est pas adressé dans ces brevets. Par ailleurs, l'étape d'extraction liquide-liquide proposée nécessite une pré-purification de l'effluent butadiène pour éliminer les composés légers, l'eau résiduelle et des composés oxygénés tel que l'acétaldéhyde, l'éthanol ou le diéthyléther.

Aujourd'hui, la principale source de butadiène est pétrolière. Ce dernier est extrait d'une coupe C₄ produite par vapocraquage de naphta, contenant entre 35 et 60 % pds de butènes/butanes, entre 30 et 60 % pds de butadiène, typiquement 0.5 à 2 % pds (parfois plus) d'acétylènes, en particulier du vinylacétylène, ainsi qu'une faible quantité de 1,2-butadiène (de l'ordre de 0.1 % pds) et de composés légers comme le propane et le propylène. L'extraction est réalisée par une distillation extractive avec un solvant polaire aprotique avec une pureté supérieure à 99.5% pds et un rendement généralement supérieur à 98 % pds. Les distillations extractives utilisant la N-méthylpyrrolidone (NMP), le diméthylformamide (DMF) et l'acétonitrile (ACN) comme solvant sont les plus utilisés et les plus répandus.

La distillation extractive appliquée aux coupes C₄ issues du vapocraquage est une alternative, mais son extrapolation aux effluents butadiène issus des procédés de production à partir d'éthanol n'est pas évidente en raison de compositions différentes, à savoir une concentration en butadiène supérieure à 80 % pds et en butènes et butanes inférieure à 15% pds, une teneur en acétylènes globalement plus faible et la présence potentielle de composés oxygénés comme l'acétaldéhyde, le diéthyl éther (DEE) et l'eau, et d'alcynes produits avec la désactivation progressive des catalyseurs.

### OBJET ET INTÉRÊT DE L'INVENTION

L'invention concerne un procédé de production de butadiène à partir d'une charge éthanol comprenant au moins 80% poids d'éthanol, ledit procédé comprenant :
a) une étape de conversion de l'éthanol en butadiène comprenant :
   - une section de séparation eau-éthanol alimentée par au moins une fraction de ladite charge éthanol et par une fraction de l'effluent éthanol-eau issu de l'étape e) et produisant un effluent éthanol et un effluent eau purgée ;
   - une section réactionnelle alimentée par ledit effluent éthanol et opérée à une pression comprise entre 0,1 et 1,0 MPa et à une température comprise entre 200 et 500°C en présence d'un catalyseur et produisant un effluent réactionnel ;
b) une étape de distillation alimentée par l'effluent réactionnel issu de l'étape a) et produisant un résidu réactifs pollués et un distillat butadiène, opérée à une pression comprise entre 0,1 et 1 MPa ;
c) une étape de lavage à l'eau comprenant au moins une section de lavage gaz-liquide alimentée en fond par le distillat butadiène issu de l'étape b) et en tête par un flux d'eau, avantageusement d'origine externe audit procédé de production de butadiène, et produisant en tête un extrait butadiène hydraté et en fond un raffinat eau usée ;
d) une étape de purification du butadiène alimentée par l'extrait butadiène issu de l'étape c) et produisant au moins un effluent gaz légers et un effluent butadiène purifié comprenant au moins :
   - une section de séparation du butène-1 par distillation extractive alimentée par ledit extrait butadiène, avantageusement comprimé, et par un flux comprenant un solvant, séparant en tête un effluent gaz légers, et en fond un résidu butadiène ;
   - une section de séparation du solvant par distillation alimentée par ledit résidu butadiène, séparant en tête un distillat butadiène étêté et en fond un résidu solvant ;
   - une section de distillation finale alimentée par le distillat butadiène étêté, séparant en tête un effluent butadiène purifié et en fond un résidu butènes ;
e) une étape de traitement des effluents alimentée par le raffinat eau usée issu de l'étape c) et par le résidu réactifs pollués issu de l'étape b) et produisant au moins un effluent éthanol-acétaldéhyde, un effluent éthanol-eau, un ou plusieurs effluents huiles brunes.

Le procédé selon l'invention permet un gain énergétique important au regard de l'art antérieur. En particulier, l'absence de traitement de la fraction vapeur de l'effluent réactionnel par lavage avec un flux d'éthanol suivi d'un lavage à l'eau et l'absence des boucles de recycles des effluents aqueux du procédé permettent de réduire les débits de circulation d'éthanol et d'eau dans les sections de traitement des effluents et, du fait de cette réduction, de soulager les dites sections.

Le procédé selon l'invention permet également de produire un butadiène aux spécifications, même lorsque des sous-produits, en particulier les butynes, sont générés en plus grande quantité du fait de la désactivation des catalyseurs mis en oeuvre dans les étapes réactionnelles, sans perte de rendement globale et avec un gain énergétique maintenu.

### DESCRIPTION DÉTAILLÉE DE L'INVENTION

### Charge

La charge éthanol utilisée dans le procédé selon l'invention peut provenir de toute origine, fossile, végétale ou animale, et en particulier des procédés de production d'éthanol à partir de ressources végétale. Ladite charge comprend au moins 80% poids d'éthanol, préférentiellement au moins 90% poids, et de manière préférée au moins 93 % poids. De manière très préférée, ladite charge éthanol répond aux spécifications d'éthanol carburant EN 15376.

### Étape a) de conversion de l'éthanol en butadiène

Le procédé selon l'invention comprend une étape a) de conversion de l'éthanol en butadiène.

Ladite étape a) comprend une section de séparation eau-éthanol alimentée par au moins une fraction de ladite charge éthanol et par une fraction de l'effluent éthanol-eau issu de l'étape e) et produisant un effluent éthanol et un effluent eau purgée.

L'effluent éthanol issu de ladite section est constitué majoritairement d'éthanol. Par majoritairement, on entend plus de 80% poids, de préférence plus de 84 % poids. De manière non limitative, l'effluent riche en éthanol issu de ladite section peut contenir des impuretés comme de l'eau, de l'acétate d'éthyle, du butanol et de l'hexanol. Les impuretés autre que l'eau représentent préférentiellement moins de 10%, de façon privilégiée moins de 5%, de façon encore plus préférentielle moins de 2% poids dudit effluent.

Ladite section de séparation eau-éthanol est avantageusement réalisée par distillation. Au moins une fraction de ladite charge éthanol est alors introduite en tête de ladite distillation, ce qui a pour effet faciliter la distillation de l'éthanol en présence d'impuretés, de réduire le reflux et d'augmenter la concentration en éthanol dans l'effluent éthanol alimentant la section réactionnelle et donc de baisser le débit total de cet effluent pour un même débit d'éthanol, ce qui permet de travailler avec une section réactionnelle de plus faible volume que dans l'art antérieur, dans lequel la charge éthanol est d'abord utilisée pour solubiliser le butadiène dans l'effluent vapeur de la section réactionnelle.

Cette utilisation de la charge éthanol selon l'art antérieur a pour avantage de permettre d'extraire les composés les plus légers compris dans l'effluent de la section réactionnelle, ces composés étant séparés par lavages successifs avec la charge éthanol et avec un flux d'eau. Dans le procédé selon l'invention, ces composés légers restent avec le butadiène jusqu'à l'étape de purification du butadiène, ce qui a pour conséquence d'augmenter la taille des équipements et peut, en fonction de la concentration en composés légers, imposer d'utiliser une étape de pré-lavage ou des groupes froids en tête des sections de séparation du butène-1 afin d'assurer la liquéfaction d'une partie du distillat et d'assurer un reflux. Ce choix est donc à priori néfaste aux performances du procédé. Or, la demanderesse s'est aperçue que l'alimentation de la charge éthanol en tête d'une section de séparation eau-éthanol en association avec une étape de purification du butadiène réalisée par distillation extractive et plus apte à gérer les composés légers, malgré l'envoi des composés légers dans l'étape b) de distillation (ayant à priori un impact négatif car plus énergivore) aboutissait au global à une amélioration des performances du procédé selon l'invention, en particulier par l'amélioration du fonctionnement de la section de séparation eau-éthanol et par la minimisation de réaction éthanol-acétaldéhyde dans toutes les sections où il y a présence d'éthanol et d'acétaldéhyde (boucles de recyclage).

Dans un mode de réalisation de l'invention, l'effluent éthanol subit une étape de purification avant d'être alimenté dans la section réactionnelle. Par purification, on entend mettre en contact ledit effluent avec des adsorbants comme par exemple du charbon actif, de la silice, de l'alumine ou encore une résine polymérique fonctionnalisée. Par exemple, un charbon actif permet d'éliminer les traces de butanol, de méthanol et d'hexanol.

Ladite étape a) comprend une section réactionnelle alimentée par ledit effluent éthanol et éventuellement une partie de ladite charge éthanol, et opérée à une pression comprise entre 0,1 et 1,0 MPa, de préférence entre 0,1 et 0,5 MPa, de manière préférée entre 0,1 et 0,3 MPa et à une température comprise entre 200 et 500°C en présence d'un catalyseur et produisant un effluent réactionnel.

Dans un premier arrangement particulier du procédé selon l'invention, ladite section réactionnelle comprend une zone réactionnelle, alimentée par ledit effluent éthanol et éventuellement par une partie de ladite charge éthanol, permettant de convertir l'éthanol en au moins du butadiène. Elle est opérée en présence de tout catalyseur connu de l'Homme du métier, par exemple un catalyseur de type silice avec un oxyde de magnésium, à une température avantageusement comprise entre 300 et 400°C, de manière préférée entre 320 et 370°C et à une pression avantageusement comprise entre 0,1 et 0,5 MPa, de manière préférée entre 0,1 et 0,3 MPa.

Dans un deuxième arrangement particulier du procédé selon l'invention, ladite section réactionnelle comprend deux zones réactionnelles, la première alimentée par une fraction dudit effluent éthanol et éventuellement une partie de ladite charge éthanol, permettant de convertir l'éthanol en acétaldéhyde, et la seconde alimentée par l'effluent de la première zone réactionnelle, par la fraction résiduelle dudit effluent éthanol et par une fraction de l'effluent éthanol-acétaldéhyde issu de l'étape e) et éventuellement par une partie de ladite charge éthanol, permettant de convertir le mélange d'éthanol et d'acétaldéhyde en au moins du butadiène.

Avantageusement, dans ce deuxième arrangement, un moyen de séparation gaz-liquide est mis en œuvre entre les deux zones réactionnelles pour séparer l'effluent de la première zone réactionnelle en un effluent gaz et un effluent liquide. L'effluent gaz, comprenant de l'hydrogène, peut être traité de la même manière que l'effluent hydrogène selon les étapes de traitement de l'hydrogène C1) et C2) décrites dans WO2016/042095. L'effluent liquide alimente la deuxième zone réactionnelle.

Dans ce deuxième arrangement, ladite première zone réactionnelle est opérée en présence d'un catalyseur comprenant un oxyde de cuivre, ou de tout autre catalyseur adapté bien connu de l'Homme du métier.

Le rapport molaire éthanol sur acétaldéhyde en entrée de ladite seconde zone réactionnelle est compris entre 1 et 5, de manière préférée compris entre 1 et 3,5, de manière encore plus préférée entre 2,5 et 3,5. Ladite seconde zone réactionnelle est opérée en présence d'un catalyseur, avantageusement un catalyseur supporté sur silice choisi dans le groupe constitué par les catalyseurs comprenant de l'oxyde de tantale, de zirconium ou de niobium, préférentiellement comprenant 2% d'oxyde de Tantale (voir par exemple Corson, Jones, Welling, Hincbley, Stahly, Ind. Eng Chem. 1950, 42, 2, 359-373). Ladite seconde zone réactionnelle est opérée à une température comprise entre 300 et 400°C, de manière préférée entre 320 et 370°C et à une pression comprise entre 0,1 et 1,0 MPa, de préférence entre 0,1 et 0,5 MPa, de manière préférée entre 0,1 et 0,3 MPa.

L'effluent réactionnel de ladite zone réactionnelle dans le premier arrangement particulier selon l'invention, ou de ladite seconde zone réactionnelle dans le deuxième arrangement particulier selon l'invention, comprend encore de l'éthanol, ainsi que de nombreuses impuretés produites avec le butadiène, parmi lesquelles de l'hydrogène, de l'éthylène, du propylène, du diéthyl ether (DEE), de l'acétate d'éthyle, du butanol, de l'hexanol, des butènes, des butynes, des pentènes, des pentadiènes, des hexènes, des hexadiènes, du crotonaldéhyde, du butyraldéhyde, du diéthylacétal, et de l'acide acétique. Il alimente l'étape b) de distillation.

### Etape b) de distillation

Le procédé selon l'invention comprend une étape b) de distillation alimentée par l'effluent réactionnel issu de l'étape a) et produisant un résidu réactifs pollués et un distillat butadiène.

L'effluent réactionnel issu de l'étape a) alimente ladite étape b) de distillation de manière à séparer en tête un distillat butadiène comprenant la majorité du butadiène, et en fond un résidu réactifs pollués. Par la majorité, on entend plus de 80% poids du butadiène compris dans l'alimentation de ladite étape de distillation, préférentiellement plus de 90%, de manière préférée plus de 95%, de manière encore plus préférée plus de 98%, de manière très préférée plus de 99% et de manière très avantageuse plus de 99,5% poids du butadiène compris dans ladite alimentation de ladite étape.

Ce résidu réactifs pollués comprend de l'éthanol et de l'acétaldéhyde, et comprend également de l'eau et des sous-produits formés dans l'étape a), comme par exemple le diéthyl éther l'acétate d'éthyle et les huiles brunes. Ledit résidu réactifs pollués alimente ensuite l'étape e) de traitement des effluents. Ladite étape b) de distillation est opérée à une pression comprise entre 0,1 et 1 MPa et de manière préférée entre 0,2 et 0,5 MPa.

L'effluent réactionnel issu de l'étape a) subit avantageusement une séparation gaz-liquide avant d'alimenter l'étape b). Cette séparation consiste à refroidir l'effluent réactionnel issu de l'étape a) à une température comprise entre 10 et 100°C, de préférence entre 25 et 80°C, pour condenser une partie dudit effluent réactionnel et obtenir, dans un séparateur gaz/liquide, au moins un effluent liquide et au moins un effluent vapeur. Cette section peut avantageusement être opérée avec plusieurs étages de refroidissement et de séparation gaz/liquide avec éventuellement la compression des effluents vapeur entre deux étages. L'effluent vapeur alimente alors l'étape b) de distillation en tant qu'effluent réactionnel issu de l'étape a). L'effluent liquide alimente l'étape e) de traitement des effluents.

### Etape c) de lavage à l'eau

Le procédé selon l'invention comprend une étape c) de lavage à l'eau alimentée par un flux d'eau et par le distillat butadiène issu de l'étape b) et produisant un extrait butadiène hydraté et un raffinat eau usée.

L'étape c) de lavage à l'eau comprend au moins une section de lavage gaz-liquide alimentée en fond par le distillat butadiène issu de l'étape b) et en tête par un flux d'eau, de préférence d'origine externe audit procédé de production de butadiène, et produisant en tête un extrait butadiène hydraté et en fond un raffinat eau usée.

Ledit raffinat eau usée contient de l'acétaldéhyde et un peu de butadiène, et alimente l'étape e) de traitement des effluents.

L'objectif de l'étape c) est d'éliminer les impuretés polaires, en particulier l'acétaldéhyde qui ne doit pas être présent au-delà de quelques ppm dans le butadiène final. Le distillat butadiène issu de b) comprend la majorité du butadiène, mais contient encore de nombreuses impuretés, dont une quantité importante d'acétaldéhyde qui forme un pincement important avec le butadiène et ne peut donc pas être complètement éliminé par distillation lors de l'étape b). Ainsi, le débit dudit flux d'eau est ajusté pour obtenir la spécification recherchée en acétaldéhyde dans l'effluent butadiène purifié issu de l'étape d). L'étape c) permet également de récupérer toutes les impuretés polaires résiduelles entrainées dans le distillat butadiène.

Ledit flux d'eau est avantageusement refroidi à une température inférieure à 25°C, de préférence inférieure à 20°C avant d'alimenter la section de lavage gaz-liquide de manière à faire le lavage avec une quantité d'eau réduite. La température d'alimentation dudit flux d'eau est choisie de manière à ne pas former d'hydrates avec le butadiène et les hydrocarbures légers encore présents dans le distillat butadiène issu de l'étape b). La pression de la colonne de lavage est déterminée de façon à assurer qu'il n'y ait aucune condensation du butadiène et qu'il reste bien sous forme gaz. La pression à cette étape est de préférence comprise entre 0,1 et 1 MPa, et de manière préférée entre 0,2 et 0,3 MPa.

### Etape d) de purification du butadiène

Le procédé selon l'invention comprend une étape d) de purification du butadiène alimentée par l'extrait butadiène issu de l'étape c) et produisant au moins un effluent gaz légers et un effluent butadiène purifié.

L'extrait butadiène hydraté issu de l'étape c) est avantageusement comprimé à une pression comprise entre 0,1 et 1,0 MPa, préférentiellement entre 0,1 et 0,7 MPa, et de manière préférée entre 0,2 et 0,5 MPa. L'effet de cette compression est principalement de diminuer le débit volumique de gaz. Il n'est pas nécessaire de refroidir l'extrait butadiène à l'issue de la compression.

Ladite étape d) est alimentée par l'extrait butadiène, avantageusement comprimé, issu de l'étape c). Ledit extrait butadiène comprend au moins 70% poids, de préférence au moins 80% pds de butadiène ainsi que des impuretés, dues en particulier à la dégradation de la sélectivité en butadiène dans la section réactionnelle, parmi lesquelles on compte des traces d'impuretés oxygénées telles que l'acétaldéhyde, le diéthyl éther et l'eau, et au plus 15% poids d'impuretés en C₄ de type butènes et butanes, des hydrocarbures comprenant au moins 5 atomes de carbone (hydrocarbures C₅⁺) ainsi que des gaz légers, en particulier de l'hydrogène, de l'éthane, de l'éthylène, du propane, du propylène.

Ladite étape d) comprend une section de séparation du butène-1 par distillation extractive alimentée par ledit extrait butadiène, avantageusement comprimé, et par un flux comprenant un solvant et séparant en tête un effluent gaz légers, et en fond un résidu butadiène.

Par solvant, on entend tout solvant polaire, miscible en phase liquide avec ladite charge butadiène dans les conditions opératoires de ladite section de séparation du butène-1, ayant une volatilité plus faible que les composés 1,3-butadiène, butène-2 et butynes, de manière à rester en phase liquide dans ladite section de séparation du butène-1, mais pouvant être séparé de ces composés par distillation. Ledit solvant est avantageusement choisi dans le groupe constitué par le diméthylformamide (DMF), la N-méthylpyrrolidone, l'acétonitrile et leurs mélanges.

Dans le cas préféré où ledit solvant est le DMF, le mélange eau-DMF étant corrosif, la teneur en eau dans l'extrait butadiène, avantageusement comprimé, devra être ajustée en accord avec les contraintes métallurgiques. De plus, les éléments corrodés auront tendance à catalyser la réaction de polymérisation du butadiène, entrainant une perte de rendement et un risque de colmatage des lignes. La teneur en eau peut être réduite par tout moyen connu de l'Homme du métier, par exemple par séchage, avantageusement par séchage sur adsorbant, l'adsorbant pouvant être silicique et/ou aluminique. De manière non limitative, cet adsorbant peut être une zéolite telle qu'une zéolite 3A ou 4A. De manière avantageuse, la teneur en eau dans l'extrait butadiène, avantageusement comprimé, est réduite à une valeur inférieure à 3% poids dudit effluent. Il est également possible d'ajouter un inhibiteur de corrosion connu de l'Homme du métier audit effluent.

L'effluent gaz légers produit en tête de la distillation extractive de ladite section de séparation du butène-1 comprend des butènes 1 et 2, ainsi que des gaz légers tels que l'hydrogène, l'éthane, l'éthylène, le propane, le propylène. Lorsque la teneur en gaz légers dans l'extrait butadiène, avantageusement comprimé, est importante, par exemple supérieure à 2% du poids total dudit extrait, un refroidissement important de la tête de ladite distillation extractive peut être nécessaire afin d'assurer un reflux suffisant dans ladite colonne. Ce refroidissement est classiquement réalisé en utilisant un groupe froid.

L'effluent gaz légers peut être brulé pour fournir une partie de la chaleur nécessaire au circuit d'huile chaude ou aux chaudières à vapeur du procédé.

De manière avantageuse, ledit extrait butadiène issu de l'étape c), avantageusement comprimé, est prélavé par mise en contact avec un flux comprenant un solvant polaire choisi dans le groupe constitué par le diméthylformamide (DMF), la N-méthylpyrrolidone (NMP) et l'acétonitrile préalablement à son alimentation dans ladite section de séparation du butène-1. Ce prélavage permet de séparer la majorité des gaz légers et ainsi de se passer de refroidissement nécessitant la mise en œuvre d'un groupe froid en tête de ladite section de séparation du butène-1.

Ladite section de séparation du butène-1 par distillation extractive est opérée de telle sorte que ledit résidu butadiène comprenne au moins 95% poids, avantageusement au moins 98% poids et de manière préférée au moins 99% poids du butadiène compris dans ledit extrait butadiène, avantageusement comprimé,. Ladite section est également opérée de telle sorte que la quantité de butène-1 dans ledit résidu butadiène représente au plus 0,5% du poids de butadiène compris dans ledit résidu. L'opération est effectuée en ajustant le ratio débit de solvant sur débit d'effluent butadiène, ainsi que le taux de reflux de la distillation extractive, comme connu de l'Homme du métier.

Ladite section de séparation du butène-1 est opérée à la pression la plus basse possible pour limiter l'exposition des flux riches en butadiène à des hautes températures où une polymérisation ou décomposition pourraient avoir lieu. De préférence, la pression d'opération de ladite section est inférieure à 0,6 MPa, et de manière préférée inférieure à 0,5 MPa.

Ladite étape d) comprend une section de séparation du solvant par distillation alimentée par ledit résidu butadiène issu de la section de séparation du butène-1 et séparant en tête un distillat butadiène étêté et en fond un résidu solvant.

La distillation est avantageusement une distillation classique connue de l'Homme du métier, opérée de telle sorte que ledit résidu solvant comprenne moins de 1% poids de butadiène, avantageusement ne comprenne plus de butadiène, et que ledit distillat butadiène étêté comprenne moins de 0,5% poids de solvant, avantageusement ne comprenne plus de solvant.

Ladite distillation est avantageusement opérée à la pression la plus basse possible pour limiter l'exposition des flux riches en butadiène à des hautes températures où une polymérisation ou décomposition pourraient avoir lieu. Ladite distillation est avantageusement opérée à une température en tête inférieure à 60°C, de préférence inférieure à 50°C, et à une pression en tête inférieure à 0,5 MPa, de préférence inférieure à 0,4 MPa.

Ledit résidu solvant alimente avantageusement la section de séparation du butène-1 en tant que flux comprenant un solvant, avantageusement en mélange avec un appoint de solvant.

Ladite étape d) comprend une section de distillation finale alimentée par le distillat butadiène étêté issu de la section de séparation du solvant, séparant en tête un effluent butadiène purifié et en fond un résidu butènes.

Cette section permet d'éliminer les impuretés lourdes, et notamment les traces de cis-2-butènes, ainsi que les butynes-1 résiduels et le 1,2-butadiène et diéthyléther éventuellement présents dans le distillat butadiène étêté.

Ledit effluent butadiène purifié comprend au moins 99,5 % poids de butadiène. Le rendement de l'étape d) de purification selon l'invention, défini comme le débit de butadiène dans l'effluent butadiène purifié sur le débit de butadiène dans l'extrait butadiène, avantageusement comprimé, alimentant l'étape d) est au moins égal à 95 % poids, de préférence à 98%, de manière préférée supérieur à 99% poids

Les alcynes à 4 atomes de carbone, éventuellement présents dans le distillat butadiène étêté alimentant ladite section de distillation finale sont séparés dans l'effluent butadiène purifié. Si la distillation finale ne permet pas de séparer les alcynes à 4 atomes de carbone, éventuellement présents dans le distillat butadiène étêté, de manière à ce que leur teneur dans ledit effluent butadiène purifié réponde aux spécifications requises pour une utilisation ultérieure dudit effluent, on procède avantageusement au traitement dudit distillat butadiène étêté, préalablement à son alimentation dans ladite section de distillation finale, dans une seconde section de distillation extractive.

Ladite seconde section de distillation extractive est alimentée par ledit distillat butadiène étêté et par un flux comprenant un solvant et produit en tête le distillat butadiène étêté alimentant la section de distillation finale et en fond un résidu solvant usé comprenant le solvant et, en fonction de leur présence, des alcynes à 4 atomes de carbone.

Ledit solvant est avantageusement choisi dans le groupe constitué par le diméthylformamide (DMF), la N-méthylpyrrolidone (NMP) et l'acétonitrile.

Ladite seconde section de distillation extractive est opérée de telle sorte que ledit distillat butadiène étêté alimentant la section de distillation finale comprenne au moins 98% poids, avantageusement au moins 99% poids du butadiène compris dans ledit distillat butadiène alimentant ladite seconde section de distillation extractive. Ladite section est également opérée de telle sorte que la teneur en alcynes à 4 atomes de carbones présente dans l'effluent butadiène purifié en tête de la section de distillation finale soit conforme aux spécifications exigées pour l'utilisation ultérieure dudit effluent. L'opération est effectuée en ajustant le ratio débit de solvant sur débit de distillat butadiène étêté, ainsi que le taux de reflux de ladite section de distillation extractive, comme connu de l'Homme du métier.

Ladite seconde section de distillation extractive est opérée à la pression la plus basse possible pour limiter l'exposition des flux riches en butadiène à des hautes températures où une polymérisation ou décomposition pourraient avoir lieu. De préférence, la pression d'opération de ladite section est inférieure à 0,6 MPa, et de manière préférée inférieure à 0,5 MPa.

Dans l'arrangement particulier où une seconde section de distillation extractive est mise en œuvre, ledit résidu solvant usé alimente une seconde section de séparation du solvant par distillation séparant en tête un distillat alcynes à 4 atomes de carbone et en fond un résidu solvant.

La distillation est une distillation classique connue de l'Homme du métier, opérée de telle sorte que ledit résidu solvant comprenne moins de 1% poids de butadiène, avantageusement ne comprenne plus de butadiène, et que la perte en solvant dans ledit distillat alcynes à 4 atomes de carbone soit inférieure à 0,05% poids, de préférence inférieure à 0,01% poids. Par perte en solvant, on entend le ratio du débit de solvant dans ledit distillat alcynes à 4 atomes de carbone sur le débit de solvant dans ledit résidu solvant usé.

Ladite distillation est également opérée de telle sorte que la teneur en composés acétyléniques dans ledit distillat alcynes à 4 atomes de carbone ne dépasse pas 30% poids pour éviter tout risque d'explosion favorisé par une augmentation de la pression et de la température, phénomène connu de l'Homme du métier.

Ladite distillation est avantageusement opérée à une température en tête inférieure à 60°C, de préférence inférieure à 50°C, et à une pression en tête inférieure à 0,5 MPa, de préférence inférieure à 0,4 MPa.

Ledit résidu solvant alimente avantageusement la seconde section de distillation extractive en tant que flux comprenant un solvant, avantageusement en mélange avec un appoint de solvant.

Le résidu solvant issu de la section de séparation du solvant et le résidu solvant issu de la seconde section de séparation du solvant peuvent avantageusement être mélangés avant d'alimenter la section de séparation du butène-1 et la seconde section de distillation extractive. Tout ou partie du résidu solvant issu de la section de séparation du solvant et éventuellement de la seconde section de séparation du solvant peut avantageusement être envoyé dans une section de purification du solvant, par exemple par distillation ou tout autre opération connu de l'Homme du Métier, pour séparer les impuretés lourdes.

### Etape e) de traitement des effluents

Le procédé selon l'invention comprend une étape e) de traitement des effluents alimentée par le raffinat eau usée issu de l'étape c) et par le résidu réactifs pollués issu de l'étape b) et produisant au moins un effluent éthanol-acétaldéhyde, un effluent éthanol-eau, un ou plusieurs effluents huiles brunes, de préférence un effluent huiles brunes légères et un effluent huiles brunes lourdes.

De préférence, ladite étape e) comprend au moins une section de lavage/contre-lavage, une section de distillation des huiles brunes légères, une section de distillation des huiles brunes lourdes et une section de séparation de l'acétaldéhyde.

Ladite section de lavage/contre-lavage préférentielle est alimentée en un point intermédiaire par ledit résidu réactifs pollués issu de l'étape b).

Ladite section de lavage/contre-lavage préférentielle est alimentée en fond par un effluent hydrocarbures et en tête par au moins une fraction du raffinat eau usée issue de l'étape c). L'effluent hydrocarbures et la fraction du raffinat eau usée issue de l'étape c) sont alimentés à une température de préférence comprise entre 10 et 70°C, préférentiellement entre 45 et 55°C. Ladite section de lavage/contre-lavage produit en tête un extrait hydrocarbures de lavages chargé d'une fraction des impuretés et des huiles brunes, et en fond un raffinat éthanol / acétaldéhyde / eau.

Ladite section de lavage/contre-lavage est de préférence opérée à une pression comprise entre 0,1 et 0,5 MPa, préférentiellement entre 0,2 et 0,4 MPa. De préférence, l'ajout d'eau pour réaliser le contre-lavage est tel que la teneur en eau dans le raffinat eau / éthanol / acétaldéhyde est supérieure à 30% poids, de manière préférée supérieur à 40% poids.

Dans un mode de réalisation, le contact entre les deux phases liquides dans ladite section de lavage/contre-lavage est réalisé au sein d'un extracteur liquide-liquide. Différents modes de contact peuvent être envisagés. On peut citer de manière non limitative, une colonne garnie, une colonne puisée, ou bien une colonne compartimentée agitée. Dans un autre mode de réalisation, le contact entre les deux phases liquides dans ladite section de lavage/contre-lavage est réalisé au sein d'un contacteur membranaire, ou une cascade de contacteurs membranaires. Ce mode de contact est particulièrement bien adapté au système mis en œuvre. En effet, les mélanges eau-éthanol-hydrocarbures sont connus pour former des émulsions stables, qui peuvent être problématiques dans un extracteur liquide-liquide. Le contacteur membranaire permet de générer une aire de contact importante, favorisant le transfert des impuretés et des huiles vers la phase hydrocarbure, sans générer d'émulsion.

Les procédés de l'art antérieur proposent un contre-lavage avec un flux d'eau recyclé. Or, la demanderesse a découvert que l'opération de contre-lavage est plus efficace avec de l'eau contenant que très peu d'acétaldéhyde et de butadiène qu'avec une eau recyclée qui peut être polluée par des impuretés liquides, et ce sans augmenter le débit total d'eau tant en entrée qu'en sortie du procédé.

L'utilisation du raffinat eau usée issu de l'étape c) limite l'accumulation d'impuretés par rapport aux procédés de l'art antérieur. De plus, l'injection de la charge éthanol au niveau de la section de séparation eau-éthanol et sa non-utilisation pour le lavage de l'effluent réactionnel limite la concentration en éthanol dans les boucles de circulation de l'étape e) de traitement des effluents, limitant ainsi les réactions entre éthanol et acétaldéhyde dans la section de lavage-contre lavage, ce qui limite les pertes sous forme d'hemi et di-éthyl acétal, sans influencer négativement le fonctionnement des sections de distillation des huiles brunes légères et lourdes.

Ledit extrait hydrocarbures de lavages alimente ladite section de distillation des huiles brunes légères, laquelle produit en tant que distillat ledit effluent huiles brunes légères, et un résidu hydrocarbures comprenant la fraction lourde des huiles brunes.

Ledit effluent huiles brunes légères est composé d'impuretés produites dans la section réactionnelle de l'étape a), principalement du diéthyl éther, d'acétate d'éthyle et du crotonaldéhyde, ainsi que de la fraction légère des huiles brunes, composée d'impuretés en quantité plus faible, parmi lesquelles du pentène, de l'isoprène, du butanal, du vinyl ethyl ether. Cet effluent peut être brulé pour fournir une partie de la chaleur nécessaire au circuit d'huile chaude ou aux chaudières à vapeur du procédé, ou distillé pour récupérer un effluent diéthyl éther et/ou un effluent acétate d'éthyle/crotonaldéhyde, qui pourra être soit valorisée, soit recyclée dans la section réactionnelle de l'étape a) pour être retransformé.

Ledit résidu hydrocarbures contient essentiellement les hydrocarbures servant au lavage, mais également la fraction la plus lourde des huiles brunes. Pour éviter l'accumulation des huiles brunes par le recyclage de l'effluent hydrocarbures vers l'extracteur liquide-liquide, une fraction dudit résidu hydrocarbures est traitée dans ladite section de distillation des huiles lourdes, consistant en une colonne à distiller, laquelle produit un distillat hydrocarbures composé pour l'essentiel d'hydrocarbures avec encore quelques traces d'huiles brunes et, en tant que résidu, ledit effluent huiles brunes lourdes comprenant plus de 80%, préférentiellement plus de 85% d'hydrocarbures ainsi que les huiles brunes les plus lourdes. La fraction dudit effluent hydrocarbures envoyée vers ladite section de distillation des huiles est comprise entre 5 et 30% du débit total dudit résidu hydrocarbures, et préférentiellement entre 10 et 20%. Le distillat hydrocarbures est mélangé à la fraction du résidu hydrocarbures qui n'a pas été traité dans ladite section de distillation des huiles lourdes afin de former l'effluent hydrocarbures alimentant ladite section de lavage/contre-lavage.

Cet effluent, qui représente de préférence entre 0,1 et 20% de la charge de ladite section de distillation des huiles lourdes, préférentiellement entre 0,3 et 5%, peut être brûlé pour fournir une partie de la chaleur nécessaire au circuit d'huile chaude ou aux chaudières à vapeur du procédé. Un appoint d'hydrocarbures équivalent aux pertes en fond de ladite section de distillation des huiles lourdes est nécessaire pour maintenir le débit de lavage constant. Cette colonne est réglée de manière à maintenir constante la concentration en huiles brunes dans la boucle de recyclage des hydrocarbures (boucle effluent hydrocarbures /effluent hydrocarbures de lavages).

Les effluents huiles brunes légères et lourdes sont éliminés du procédé.

Le résidu réactifs pollués issu de l'étape b) comprend principalement de l'éthanol, de l'acétaldéhyde, de l'eau mais aussi des impuretés telles que le diéthyl éther, l'acétate d'éthyle et les huiles brunes telles que définies précédemment. Ces impuretés peuvent s'accumuler si elles sont renvoyées vers l'étape a) au sein de l'effluent éthanol-acétaldéhyde et/ou de l'effluent éthanol-eau et qu'elles ne sont que partiellement converties dans la section réactionnelle de l'étape a). La section de lavage-contre lavage permet de récupérer une partie de ces impuretés avant les sections de séparation eau-acétaldéhyde de l'étape e) et de séparation eau-éthanol de l'étape a), ce qui permet d'éviter la démixtion des huiles brunes au sein de ces sections.

Le lavage du résidu réactifs pollués issu de l'étape b) avec un effluent hydrocarbures entraîne certaines impuretés, tandis que le contre-lavage du flux d'hydrocarbures avec une fraction du résidu eau usée issu de l'étape c) limite toute perte en acétaldéhyde et en éthanol.

Ledit effluent hydrocarbures peut contenir des hydrocarbures saturés et/ou insaturés et /ou aromatiques, de préférence des hydrocarbures saturés. Ledit effluent hydrocarbures est avantageusement constitué d'un mélange d'hydrocarbures ayant entre 6 et 40 atomes de carbone, de préférence entre 10 et 20 atomes de carbone. De manière non limitative, ledit effluent hydrocarbures peut être une coupe gazole ou kérosène désulfurée ou bien encore une coupe d'hydrocarbures produite par une unité de type Fischer-Tropsh.

L'ajout d'eau au sein de la section de lavage/contre-lavage permet un meilleur fonctionnement du procédé d'élimination des impuretés et des huiles brunes selon l'invention.

Le procédé selon l'invention évite ainsi la purge régulière d'éthanol afin d'éviter l'accumulation des huiles brunes, ce qui permet d'améliorer les performances globales du procédé.

Ledit effluent eau/éthanol/acétaldéhyde issu de la section de lavage-contre lavage alimente ladite section de séparation de l'acétaldéhyde, dans laquelle l'acétaldéhyde est séparé de manière à former un effluent éthanol-acétaldéhyde et un effluent éthanol-eau.

L'effluent éthanol-acétaldéhyde issu de l'étape e) est constitué majoritairement d'acétaldéhyde et d'éthanol. Par majoritairement, on entend que l'ensemble éthanol + acétaldéhyde représente plus de 80% pds, de préférence plus de 85 % poids dudit effluent. De manière non limitative, l'effluent éthanol-acétaldéhyde issu de l'étape e) peut contenir des impuretés comme de l'eau, de l'acétate d'éthyle, de l'acétone. Les impuretés autre que l'eau représentent moins de 10%, de façon préférentielles moins de 5% poids du flux.

Dans un mode de réalisation de l'invention, ledit effluent éthanol-acétaldéhyde subit une étape de purification avant d'être recyclés dans le reste du procédé. Par purification, on entend mettre en contact ledit effluent avec des adsorbants comme par exemple du charbon actif, de la silice, de l'alumine ou encore une résine polymérique fonctionnalisée.

### DESCRIPTION DES FIGURES

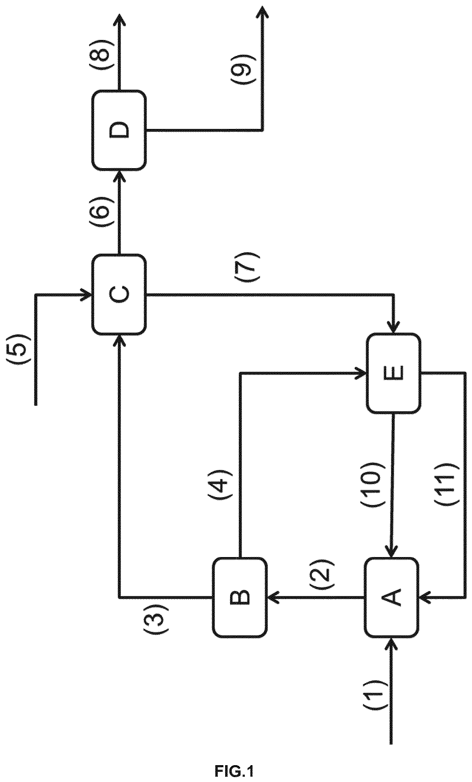

La figure 1 présente de manière schématique une vue générale du procédé selon l'invention.

Une étape de **A** de conversion de l'éthanol en butadiène est alimentée par une charge éthanol (1), et par l'effluent éthanol-eau (11) et par l'effluent éthanol-acétaldéhyde (10) issus de l'étape **E.** Cette étape produit un effluent réactionnel (2) qui alimente une étape **B** de distillation, laquelle produit un distillat butadiène (3) et un résidu réactifs pollués (4).

Le distillat butadiène (3) et un flux d'eau (5) alimentent l'étape **C** de lavage à l'eau de manière à produire un extrait butadiène hydraté (6) et un raffinat eau usée (7).

L'extrait butadiène hydraté (6) alimente l'étape **D** de purification du butadiène afin d'être séparés en un effluent gaz légers (8), un effluent butadiène purifié (9), un résidu solvant et un résidu butènes (ces deux derniers étant non représentés).

L'étape **E** de traitement des effluents est alimentée par le résidu réactifs pollués (4) issu de l'étape **B** et par le raffinat eau usée (7) issu de l'étape **C** et produit un effluent éthanol-acétaldéhyde (10), un effluent éthanol-eau (11), un effluent huiles brunes légères et un effluent huiles brunes lourdes (ces deux derniers étant non représentés).

### EXEMPLES

Les exemples suivants sont basés sur des simulations intégrant les recyclages des flux. Dans chacun des exemples, le débit de charge éthanol est ajusté de manière à obtenir une production annuelle de 150 kt/an d'un effluent butadiène purifié ayant une pureté comprise entre 99,5 et 100 % poids.

Tous les pourcentages sont des pourcentages poids.

### Exemple 1 - Comparatif

Un procédé de production de butadiène à partir d'éthanol conforme au procédé décrit dans WO2016/042095 est alimenté par 48,2 t/h d'une charge éthanol comprenant 93,4% poids d'éthanol. Ce schéma ne met pas en œuvre de distillation extractive et la charge éthanol est utilisée pour laver l'effluent réactionnel.

La charge éthanol est utilisée pour laver l'effluent vapeur issu de la section réactionnelle, après que celui-ci a été séparé de sa partie liquide et comprimé.

La charge éthanol issue de la section de lavage est mélangée à la partie liquide de l'effluent de la section réactionnelle, formant un flux de 166 t/h comprenant 54% d'éthanol, 4% d'acétaldéhyde, 23% d'eau et 11% de butadiène.

Ce flux est traité dans une section de distillation produisant en tête 20 t/h d'un distillat comprenant le butadiène et 146 t/h d'un résidu ne comprenant plus de butadiène, et comprenant 61% d'éthanol, 4% d'acétaldéhyde et 27% d'eau.

Le distillat comprenant le butadiène est traité successivement par lavage à l'eau, séchage, distillation cryogénique et séparation au DMSO afin de produire 18,75 t/h d'un effluent butadiène purifié comprenant 99,6% de butadiène. Le lavage à l'eau met en œuvre 21 t/h d'eau d'origine externe au procédé. L'eau issue du lavage du distillat comprenant le butadiène alimente la section de séparation de l'acétaldéhyde.

L'effluent vapeur lavé par la charge éthanol est lavé par de l'eau issue du traitement des effluents afin de capter les traces d'éthanol et d'acétaldéhyde. L'eau issue de ce lavage (environ 0,4 t/h) alimente une section de séparation eau-éthanol.

Les 146 t/h de résidu sont traités dans une section de lavage-contre lavage aux hydrocarbures afin de séparer les impuretés, et en particulier les huiles brunes. L'effluent de cette section, représentant 168 t/h et comprenant 53% d'éthanol, 4% d'acétaldéhyde et 40% d'eau, alimente deux colonnes à distiller permettant de séparer un effluent éthanol-acétaldéhyde, un effluent éthanol et un effluent eau, lequel est en partie (29,5 t/h) recyclé vers le lavage de l'effluent vapeur issu de la section réactionnelle (0,4 t/h) et vers la section de lavage-contre lavage (29,1 t/h), et en partie (41,4 t/h) purgé.

L'effluent éthanol (95,7 t/h comprenant 79% d'éthanol) et l'effluent éthanol-acétaldéhyde (23,2 t/h comprenant 57% d'éthanol et 27% d'acétaldéhyde) alimentent la section réactionnelle.

La consommation en énergie de ce schéma en terme d'utilités (électricité, gaz et vapeur) exprimée en MWh est de 178,8 MWh.

### Exemple 2 - Conforme à l'invention

Un procédé de production de butadiène à partir d'éthanol conforme au procédé selon l'invention est alimenté par 48,2 t/h d'une charge éthanol comprenant 93,4% poids d'éthanol.

Conformément à l'invention, la charge éthanol n'est pas utilisée pour laver l'effluent vapeur issu de la section réactionnelle. La charge éthanol est alimentée en tête de la section de séparation éthanol-eau, réalisée dans une colonne à distiller. Cette section de séparation produit en fond 41,3 t/h d'eau qui est purgée du procédé et en tête 90 t/h d'effluent éthanol alimentant la section réactionnelle.

La section réactionnelle comprend deux zones réactionnelles. La première est alimentée par 75 t/h de l'effluent éthanol. L'effluent de la première zone réactionnelle est séparé en un effluent gaz et un effluent liquide. L'effluent gaz (environ 11 t/h) est lavé par les 15 t/h de l'effluent éthanol n'alimentant pas la première zone réactionnelle. Le gaz lavé (1 t/h) comprend principalement de l'hydrogène. L'effluent éthanol ayant lavé l'effluent gaz, en mélange avec l'effluent liquide et l'effluent éthanol-acétaldéhyde issu de l'étape de traitement des effluents, alimente la seconde zone réactionnelle avec un débit total de 112 t/h.

L'effluent réactionnel issu de la seconde zone réactionnelle forme un flux de 112 t/h comprenant 39% d'éthanol, 6% d'acétaldéhyde, 28% d'eau et 17% de butadiène.

Ce flux est traité dans une section de distillation produisant en tête 20 t/h d'un distillat comprenant le butadiène et 91,5 t/h d'un résidu ne comprenant plus de butadiène, et comprenant 48% d'éthanol, 7% d'acétaldéhyde et 34% d'eau.

Le distillat comprenant le butadiène est traité successivement par lavage à l'eau et séchage, avant d'alimenter une étape de purification du butadiène par distillation extractive afin de produire 18,75 t/h d'un effluent butadiène purifié comprenant 99,6% de butadiène. Le lavage à l'eau met en œuvre 21 t/h d'eau d'origine externe au procédé. L'eau issue du lavage du distillat comprenant le butadiène alimente en tête la section de lavage-contre lavage aux hydrocarbures de l'étape de traitement des effluents (dans l'exemple non-conforme, cette eau alimente la section de séparation de l'acétaldéhyde).

Les 91,5 t/h de résidu ne comprenant plus de butadiène alimentent en un point intermédiaire une section de lavage-contre lavage aux hydrocarbures dans une étape de traitement des effluents afin de séparer les impuretés, et en particulier les huiles brunes. L'effluent de cette section, représentant 106 t/h et comprenant 41% d'éthanol, 6% d'acétaldéhyde et 49% d'eau, alimente une section de séparation de l'acétaldéhyde permettant de séparer un effluent éthanol-acétaldéhyde et un effluent éthanol-eau. L'effluent éthanol-acétaldéhyde (23,3 t/h comprenant 56% d'éthanol et 27% d'acétaldéhyde) alimente la seconde zone réactionnelle.

L'effluent éthanol-eau alimente la section de séparation éthanol-eau en un point intermédiaire, section par ailleurs alimentée en tête par la charge éthanol comme indiqué en début d'exemple.

Par rapport au procédé non-conforme, un rendement global en butadiène similaire est obtenu (même débit d'effluent butadiène purifié pour un même débit de charge).

La consommation en énergie de ce schéma en terme d'utilités (électricité, gaz et vapeur) exprimée en MWh est de 146,3 MWh, soit une réduction de 18% de la consommation d'utilités. Ce gain est en partie dû à une meilleure gestion du circuit d'eau. La réduction du débit d'eau circulant dans le procédé induit une baisse de consommation au niveau des opérations de séparation. Le choix du point d'injection de la charge éthanol allège également le travail des opérations de séparation. La charge éthanol n'étant pas utilisée pour laver les effluents réactionnels, elle n'est plus traitée dans les étapes de traitement des effluents. Ces réductions de débit ont donc également pour conséquence de diminuer la taille des équipements.

## Revendications

1. Procédé de production de butadiène à partir d'une charge éthanol comprenant au moins 80% poids d'éthanol, ledit procédé comprenant :
a) une étape de conversion de l'éthanol en butadiène comprenant :
- une section de séparation eau-éthanol alimentée par au moins une fraction de ladite charge éthanol et par une fraction de l'effluent éthanol-eau issu de l'étape e) et produisant un effluent éthanol et un effluent eau purgée ;
- une section réactionnelle alimentée par ledit effluent éthanol et opérée à une pression comprise entre 0,1 et 1,0 MPa et à une température comprise entre 200 et 500°C en présence d'un catalyseur et produisant un effluent réactionnel ;
b) une étape de distillation alimentée par l'effluent réactionnel issu de l'étape a) et produisant un résidu réactifs pollués et un distillat butadiène, opérée à une pression comprise entre 0,1 et 1 MPa ;
c) une étape de lavage à l'eau comprenant au moins une section de lavage gaz-liquide alimentée en fond par le distillat butadiène issu de l'étape b) et en tête par un flux d'eau, et produisant en tête un extrait butadiène hydraté et en fond un raffinat eau usée ;
d) une étape de purification du butadiène alimentée par l'extrait butadiène issu de l'étape c) et produisant au moins un effluent gaz légers et un effluent butadiène purifié comprenant au moins :
- une section de séparation du butène-1 par distillation extractive alimentée par ledit extrait butadiène et par un flux comprenant un solvant, séparant en tête un effluent gaz légers, et en fond un résidu butadiène ;
- une section de séparation du solvant par distillation alimentée par ledit résidu butadiène issu de la section de séparation du butène-1, séparant en tête un distillat butadiène étêté et en fond un résidu solvant ;
- une section de distillation finale alimentée par le distillat butadiène étêté issu de la section de séparation du solvant, séparant en tête un effluent butadiène purifié et en fond un résidu butènes ;
e) une étape de traitement des effluents alimentée par le raffinat eau usée issu de l'étape c) et par le résidu réactifs pollués issu de l'étape b) et produisant au moins un effluent éthanol-acétaldéhyde, un effluent éthanol-eau, un ou plusieurs effluents huiles brunes.

2. Procédé selon la revendication 1 dans lequel ladite section de séparation eau-éthanol de ladite étape a) est réalisée par distillation.

3. Procédé selon l'une des revendications précédentes dans lequel l'effluent éthanol issu de la section de séparation eau-éthanol de l'étape a) subit une étape de purification avant d'être alimenté dans la section réactionnelle.

4. Procédé selon l'une des revendications précédentes dans lequel ladite section réactionnelle de ladite étape a) comprend deux zones réactionnelles, la première alimentée par une fraction dudit effluent éthanol et éventuellement une partie de ladite charge éthanol, et la seconde alimentée par l'effluent de la première zone réactionnelle, par la fraction résiduelle dudit effluent éthanol et par une fraction de l'effluent éthanol-acétaldéhyde issu de l'étape e) et éventuellement par une partie de ladite charge éthanol, le rapport molaire éthanol sur acétaldéhyde en entrée de ladite seconde zone réactionnelle étant compris entre 1 et 5.

5. Procédé selon la revendication 4 dans lequel un moyen de séparation gaz-liquide est mis en œuvre entre les deux zones réactionnelles pour séparer l'effluent de la première zone réactionnelle en un effluent gaz et un effluent liquide, l'effluent liquide alimentant la seconde zone réactionnelle.

6. Procédé selon l'une des revendications précédentes dans lequel l'effluent réactionnel issu de l'étape a) subit une séparation gaz-liquide avant d'alimenter l'étape b) consistant à refroidir l'effluent réactionnel issu de l'étape a) à une température comprise entre 10 et 100°C et obtenir, dans un séparateur gaz/liquide, au moins un effluent liquide et au moins un effluent vapeur, l'effluent vapeur alimentant l'étape b) de distillation en tant qu'effluent réactionnel issu de l'étape a), l'effluent liquide alimentant l'étape e) de traitement des effluents.

7. Procédé selon l'une des revendications précédentes dans lequel l'extrait butadiène hydraté issu de l'étape c) est comprimé à une pression comprise entre 0,1 et 1,0 MPa.

8. Procédé selon l'une des revendications précédentes dans lequel le solvant de ladite section de séparation du butène-1 de ladite étape d) est choisi dans le groupe constitué par le diméthylformamide, la N-méthylpyrrolidone, l'acétonitrile et leurs mélanges.

9. Procédé selon l'une des revendications précédentes dans lequel, ledit extrait butadiène issu de l'étape c) est prélavé par mise en contact avec un flux comprenant un solvant polaire choisi dans le groupe constitué par le diméthylformamide (DMF), la N-méthylpyrrolidone (NMP) et l'acétonitrile préalablement à son alimentation dans ladite section de séparation du butène-1.

10. Procédé selon l'une des revendications précédentes dans lequel ledit résidu solvant de la section de séparation du solvant de l'étape d) alimente la section de séparation du butène-1 de l'étape d) en tant que flux comprenant un solvant.

11. Procédé selon l'une des revendications précédentes dans lequel le distillat butadiène étêté est traité, préalablement à son alimentation dans la section de distillation finale de l'étape d), dans une seconde section de distillation extractive, ladite seconde section de distillation extractive étant alimentée par ledit distillat butadiène étêté et par un flux comprenant un solvant et produisant en tête le distillat butadiène étêté alimentant la section de distillation finale de l'étape d) et en fond un résidu solvant usé, ledit résidu solvant usé alimentant une seconde section de séparation du solvant par distillation séparant en tête un distillat alcynes à 4 atomes de carbone et en fond un résidu solvant.

12. Procédé selon la revendication 11 dans lequel ledit résidu solvant issu de la seconde section de séparation du solvant alimente la seconde section de distillation extractive en tant que flux comprenant un solvant.

13. Procédé selon la revendication 12 dans lequel le résidu solvant issu de la section de séparation du solvant et le résidu solvant issu de la seconde section de séparation du solvant sont mélangés avant d'alimenter la section de séparation du butène-1 et la seconde section de distillation extractive.

## Patentansprüche

1. Verfahren zur Herstellung von Butadien aus einem Ethanol-Einsatzstoff, der mindestens 80 Gewichts-% Ethanol umfasst, wobei das Verfahren umfasst:
a) einen Schritt zur Umsetzung von Ethanol zu Butadien, umfassend:
- einen Abschnitt zur Wasser-Ethanol-Trennung, dem mindestens ein Anteil des Ethanol-Einsatzstoffs und ein Anteil des Ethanol-Wasser-Abstroms aus Schritt e) zugeführt werden und in dem ein Ethanolabstrom und ein abgelassener Wasserabstrom erzeugt werden;
- einen Reaktionsabschnitt, dem der Ethanolabstrom zugeführt wird und der bei einem Druck zwischen 0,1 und 1,0 MPa und bei einer Temperatur zwischen 200 und 500°C in Gegenwart eines Katalysators betrieben wird und in dem ein Reaktionsabstrom erzeugt wird;
b) einen Destillationsschritt, dem der Reaktionsabstrom aus Schritt a) zugeführt wird, in dem ein Rückstand aus verunreinigten Reaktanten und ein Butadiendestillat erzeugt werden und der bei einem Druck zwischen 0,1 und 1 MPa betrieben wird;
c) einen Schritt zum Spülen mit Wasser, der mindestens einen Gas-Flüssigkeits-Spülabschnitt umfasst, dem am Boden das Butadiendestillat aus Schritt b) und am Kopf ein Wasserstrom zugeführt werden und in dem am Kopf ein hydratisiertes Butadien als Extrakt und am Boden Abwasser als Raffinat erzeugt werden;
d) einen Schritt zur Reinigung von Butadien, dem das Butadien-Extrakt aus Schritt c) zugeführt wird und in dem mindestens ein Abgasstrom aus leichten Gasen und ein Abstrom aus gereinigtem Butadien erzeugt werden, der mindestens Folgendes umfasst:
- einen Abschnitt zur Abtrennung von Buten-1 durch extraktive Destillation, dem das Butadien-Extrakt und ein Strom zugeführt werden, der ein Lösungsmittel umfasst, in dem am Kopf ein Abgasstrom aus leichten Gasen und am Boden ein Butadienrückstand abgetrennt werden;
- einen Abschnitt zur Abtrennung des Lösungsmittels durch Destillation, dem der Butadienrückstand aus dem Abschnitt zur Abtrennung von Buten-1 zugeführt wird und in dem am Kopf ein abdestilliertes leichtes Butadiendestillat und am Boden ein Lösungsmittelrückstand abgetrennt werden;
- einen Enddestillationsabschnitt, dem das abdestillierte leichte Butadiendestillat aus dem Abschnitt zur Abtrennung des Lösungsmittels zugeführt wird und in dem am Kopf ein Abstrom aus gereinigtem Butadien und am Boden ein Rückstand aus Butenen abgetrennt werden;
e) einen Abstrom-Behandlungsschritt, dem das Abwasser-Raffinat aus Schritt c) und der Rückstand aus verunreinigten Reaktanten aus Schritt b) zugeführt werden und in dem mindestens ein Ethanol-Acetaldehyd-Abstrom, ein Ethanol-Wasser-Abstrom, ein oder mehrere Braunöl-Abströme erzeugt werden.

2. Verfahren nach Anspruch 1, wobei der Abschnitt zur Wasser-Ethanol-Trennung von Schritt a) mittels Destillation durchgeführt wird.

3. Verfahren nach einem der vorhergehenden Ansprüche, wobei der Ethanol-Abstrom aus dem Abschnitt zur Wasser-Ethanol-Trennung von Schritt a) einen Reinigungsschritt durchläuft, bevor er dem Reaktionsabschnitt zugeführt wird.

4. Verfahren nach einem der vorhergehenden Ansprüche, wobei der Reaktionsabschnitt von Schritt a) zwei Reaktionszonen umfasst, wobei der ersten Zone ein Anteil des Ethanol-Abstroms und gegebenenfalls ein Teil des Ethanol-Einsatzstoffs zugeführt werden und wobei der zweiten Zone der Abstrom der ersten Reaktionszone, der Restanteil des Ethanol-Abstroms und ein Anteil des Ethanol-Acetaldehyd-Abstroms aus Schritt e) und gegebenenfalls ein Teil des Ethanol-Einsatzstoffs zugeführt werden, wobei das Molverhältnis Ethanol zu Acetaldehyd am Eintritt der zweiten Reaktionszone zwischen 1 und 5 beträgt.

5. Verfahren nach Anspruch 4, wobei ein Mittel zur Gas-Flüssigkeits-Trennung zwischen den beiden Reaktionszonen vorgesehen ist, damit der Abstrom der ersten Reaktionszone in einen Abgasstrom und einen flüssigen Abstrom getrennt wird, wobei der flüssige Abstrom der zweiten Reaktionszone zugeführt wird.

6. Verfahren nach einem der vorhergehenden Ansprüche, wobei der Reaktionsabstrom aus Schritt a) eine Gas-Flüssigkeits-Trennung durchläuft, bevor er Schritt b) zugeführt wird, die darin besteht, den Reaktionsabstrom aus Schritt a) auf eine Temperatur zwischen 10 und 100°C abzukühlen und, in einem Gas-Flüssigkeits-Separator, mindestens einen flüssigen Abstrom und mindestens einen abströmenden Dampf zu erhalten, wobei der abströmende Dampf Destillationsschritt b) als Reaktionsabstrom aus Schritt a) zugeführt wird, wobei der flüssige Abstrom dem Abstrom-Behandlungsschritt e) zugeführt wird.

7. Verfahren nach einem der vorhergehenden Ansprüche, wobei das hydratisierte Butadien-Extrakt aus Schritt c) bei einem Druck zwischen 0,1 und 1,0 MPa verdichtet wird.

8. Verfahren nach einem der vorhergehenden Ansprüche, wobei das Lösungsmittel des Abschnitts zur Abtrennung von Buten-1 von Schritt d) aus der Gruppe ausgewählt ist, die aus Dimethylformamid, N-Methylpyrrolidon, Acetonitril und ihren Mischungen besteht.

9. Verfahren nach einem der vorhergehenden Ansprüche, wobei das Butadien-Extrakt aus Schritt c) vorgespült wird, indem es mit einem Strom in Kontakt gebracht wird, der ein polares Lösungsmittel umfasst, das aus der Gruppe ausgewählt ist, die aus Dimethylformamid (DMF), N-Methylpyrrolidon (NMP) und Acetonitril besteht, bevor es dem Abschnitt zur Abtrennung von Buten-1 zugeführt wird.

10. Verfahren nach einem der vorhergehenden Ansprüche, wobei der Lösungsmittelrückstand von dem Abschnitt zur Abtrennung des Lösungsmittels von Schritt d) dem Abschnitt zur Abtrennung von Schritt d) als Strom zugeführt wird, der ein Lösungsmittel umfasst.

11. Verfahren nach einem der vorhergehenden Ansprüche, wobei das abdestillierte leichte Butadiendestillat vor der Zuführung in den Enddestillationsabschnitt von Schritt d) in einem zweiten Abschnitt zur extraktiven Destillation behandelt wird, wobei dem zweiten Abschnitt zur extraktiven Destillation das abdestillierte leichte Butadiendestillat und ein Strom zugeführt werden, der ein Lösungsmittel umfasst, und darin am Kopf das abdestillierte leichte Butadiendestillat, das dem Enddestillationsabschnitt von d) zugeführt wird, und am Boden ein Rückstand aus verbrauchtem Lösungsmittel erzeugt werden, wobei der Rückstand aus verbrauchtem Lösungsmittel einem zweiten Abschnitt zur Abtrennung des Lösungsmittels mittels Destillation zugeführt wird, in dem am Kopf ein Destillat aus Alkinen mit 4 Kohlenstoffatomen und am Boden ein Lösungsmittelrückstand abgetrennt werden.

12. Verfahren nach Anspruch 11, wobei der Lösungsmittelrückstand aus dem zweiten Abschnitt zur Abtrennung des Lösungsmittels dem zweiten Abschnitt zur extraktiven Destillation als Strom zugeführt wird, der ein Lösungsmittel umfasst.

13. Verfahren nach Anspruch 12, wobei der Lösungsmittelrückstand aus dem Abschnitt zur Abtrennung des Lösungsmittels und der Lösungsmittelrückstand aus dem zweiten Abschnitt zur Abtrennung des Lösungsmittels gemischt werden, bevor sie dem Abschnitt zur Abtrennung von Buten-1 und dem zweiten Abschnitt zur extraktiven Destillation zugeführt werden.

## Claims

1. Process for producing butadiene from an ethanol feedstock comprising at least 80% by weight of ethanol, said process comprising:
a) a step for converting the ethanol into butadiene, comprising:
- a water-ethanol separation section fed with at least a fraction of said ethanol feedstock and with a fraction of the ethanol-water effluent obtained from step e) and producing an ethanol effluent and a purged water effluent;
- a reaction section fed with said ethanol effluent and operated at a pressure of between 0.1 and 1.0 MPa and at a temperature of between 200 and 500°C in the presence of a catalyst and producing a reaction effluent;
b) a distillation step fed with the reaction effluent obtained from step a) and producing a polluted reagent residue and a butadiene distillate, operated at a pressure of between 0.1 and 1 MPa;
c) a step of washing with water comprising at least one gas-liquid washing section fed at the bottom with the butadiene distillate obtained from step b) and at the top with a stream of water, and producing at the top a hydrated butadiene extract and at the bottom a spent water raffinate;
d) a butadiene purification step fed with the butadiene extract obtained from step c) and producing at least one light gas effluent and a purified butadiene effluent comprising at least:
- a section for separating the 1-butene by extractive distillation fed with said butadiene extract and with a stream comprising a solvent, separating at the top a light gas effluent and, at the bottom, a butadiene residue;
- a section for separating out the solvent by distillation fed with said butadiene residue obtained from the 1-butene separation section and separating at the top a topped butadiene distillate and at the bottom a solvent residue;
- a final distillation section fed with the topped butadiene distillate obtained from the solvent separation section, separating at the top a purified butadiene effluent and at the bottom a butene residue;
e) an effluent treatment step fed with the spent water raffinate obtained from step c) and with the polluted reagent residue obtained from step b) and producing at least one ethanol-acetaldehyde effluent, an ethanol-water effluent and one or more brown oil effluents.

2. Process according to Claim 1, wherein said water-ethanol separation section of said step a) is operated by distillation.

3. Process according to either of the preceding claims, wherein the ethanol effluent obtained from the water-ethanol separation section of step a) undergoes a purification step before being fed into the reaction section.

4. Process according to one of the preceding claims, wherein said reaction section of said step a) comprises two reaction zones, the first fed with a fraction of said ethanol effluent and optionally a part of said ethanol feedstock, and the second fed with the effluent from the first reaction zone, with the residual fraction of said ethanol effluent and with a fraction of the ethanol-acetaldehyde effluent obtained from step e) and optionally with a part of said ethanol feedstock, the ethanol/acetaldehyde mole ratio at the inlet of said second reaction zone being between 1 and 5.

5. Process according to Claim 4, wherein a gas-liquid separation means is used between the two reaction zones to separate the effluent from the first reaction zone into a gaseous effluent and a liquid effluent, the liquid effluent feeding the second reaction zone.

6. Process according to one of the preceding claims, wherein the reaction effluent obtained from step a) undergoes a gas-liquid separation before feeding step b) consisting in cooling the reaction effluent obtained from step a) to a temperature of between 10 and 100°C and to obtain, in a gas/liquid separator, at least one liquid effluent and at least one vapour effluent, the vapour effluent feeding the distillation step b) as reaction effluent obtained from step a), the liquid effluent feeding the effluent treatment step e).

7. Process according to one of the preceding claims, wherein the hydrated butadiene extract obtained from step c) is compressed to a pressure of between 0.1 and 1.0 MPa.

8. Process according to one of the preceding claims, wherein the solvent of said 1-butene separation section of said step d) is chosen from the group constituted by dimethylformamide, N-methylpyrrolidone and acetonitrile, and mixtures thereof.

9. Process according to one of the preceding claims, wherein said butadiene extract obtained from step c) is prewashed by placing in contact with a stream comprising a polar solvent chosen from the group constituted by dimethylformamide (DMF), N-methylpyrrolidone (NMP) and acetonitrile, prior to being fed into said 1-butene separation section.

10. Process according to one of the preceding claims,
wherein said solvent residue from the solvent separation section of step d) feeds the 1-butene separation section of step d) as a stream comprising a solvent.

11. Process according to one of the preceding claims,
wherein the topped butadiene distillate is treated, prior to being fed into the final distillation section of step d), in a second extractive distillation section, said second extractive distillation section being fed with said topped butadiene distillate and with a stream comprising a solvent and producing at the top the topped butadiene distillate feeding the final distillation section of step d), and at the bottom a spent solvent residue, said spent solvent residue feeding a second section for separating out the solvent by distillation, separating at the top a distillate of alkynes containing 4 carbon atoms and at the bottom a solvent residue.

12. Process according to Claim 11, wherein said solvent
residue obtained from the second solvent separation section feeds the second extractive distillation section as a stream comprising a solvent.

13. Process according to Claim 12, wherein the solvent
residue obtained from the solvent separation section and the solvent residue obtained from the second solvent separation section are mixed before feeding the 1-butene separation section and the second extractive distillation section.
